# EUROPEAN PATENT SPECIFICATION

(11) **EP 3 027 965 B1**
(45) Date of publication and mention of the grant of the patent: **29.04.2020**
(21) Application number: 14832569.9
(22) Date of filing: 30.07.2014
(51) Int. Cl.: F21V 9/00, G01N 31/22, G01N 21/78, G01N 33/18

(54) **ASSAY COMPOSITION, KIT AND METHOD FOR BORATE DETECTION**
ASSAY ZUSAMMENSETZUNG, KIT UND VERFAHREN ZUM BORATNACHWEIS
COMPOSITION DE DOSAGE, KIT ET PROCÉDÉ DE DOSAGE DU BORATE

(30) Priority: 30.07.2013 US 201361860220 P; 25.03.2014 US 201461970194 P
(43) Date of publication of application: 08.06.2016
(73) Proprietor: Water Lens LLC, Houston, TX 77027 (US)
(72) Inventor: DRAGNA, Justin M., Austin, TX 78754 (US); WEST, Tyler, Austin, TX 78751 (US)
(74) Representative: Uexküll & Stolberg
(86) International application number: PCT/US2014/048852
(87) International publication number: WO 2015/017524

(56) References cited:
- WO-A2-2013/012924
- JP-A- 2003 240 766
- US-A- 4 367 072
- US-A- 5 633 144
- US-A1- 2009 221 061
- CHIMPALEE N ET AL: "Flow-injection spectrofluorimetric determination of boron using Alizarin red S in aqueous solution", ANALYTICA CHIMICA ACTA, ELSEVIER, AMSTERDAM, NL, vol. 282, no. 3, 29 October 1993 (1993-10-29), pages 643-646, XP026594630, ISSN: 0003-2670, DOI: 10.1016/0003-2670(93)80129-9 [retrieved on 1993-10-29]
- CAMPANA ET AL.: 'Spectrofluorimetric Determination of Boron in Soils, Plants and Natural Waters With Alizarin Red S' ANALYST vol. 117, 1992, pages 1189 - 1191, XP055313553
- CAMPANA ET AL.: 'Spectrofluorimetric Determination of Molybdenum in Vegetal Tissues and a Pharmaceutical Compound With Alizarin Red S in Micellar Medium' ANALYST vol. 119, 1994, pages 1903 - 1906, XP055313552
- TOMSHO ET AL.: 'Elucidation of the Mechanism of the Reaction between Phenylboronic Acid and a Model Diol, Alizarin Red S' THE JOURNAL OF ORGANIC CHEMISTRY vol. 77, 2012, pages 2098 - 2106, XP055313551
- SAH ET AL.: 'Techniques for boron determination and their application to the analysis of plant and soil samples' PLANT AND SOIL vol. 193, 1997, pages 15 - 33, XP055313550

## Description

### FIELD OF THE INVENTION

This invention relates to environmental chemistry and quantitative chemical analysis.

### DESCRIPTION OF RELATED ART

Campana et al Analyst July 1992, Vol. 117 describes a spectrofluoremetric method for the determination of boron in soils, plants and natural waters with Alizarin Red S. The method employs a spectfluorometer for the fluorometric detection. The method measures the fluorescence excitation and emission spectra of the Boron-Alizarin RedS complex to determine boron concentration.

Campana et al. Analyst August 1994, Vol. 119 describes a method for the spectrofluoremetric determination of molybdenum with Alizarin Red. S in the presence of hexadecyltrimethylammonium bromide. The method measures the fluorescence excitation and emission spectra of the MO-ARS complex.

Arimori et al., Chemical Communications 2001, 2018-2019 describes fluorescent sensors for boronic and boric acids. The sensors comprise anthracenic tertiary amines as sensor molecules.

Villamil-Ramos and Yatsimirsky Chemical Communications 2011, 2694-2696 describe a method for the fluorometric detection of pyrophosphate by interaction with alizarin red S-dimethyltin(IV) complex. The detection method measures pyrophosphate dimethyltin(IV)-ARS complexes by the fluorescence at 610 nm

Tomsho and Benkovic, The Journal of Organic Chemistry 2012, Vol. 77, 2098-2106 describes the mechanism of the reaction between phenylboronic acid and Alizarin Red S. Boronic acid, or a boronate anion form a boronic ester with a 1,2-diol, whose fluorescence may be measured.

### SUMMARY

The present invention relates to a kit and an assay for the determination of dissolved borate concentration comprising a catechol dye, a multivalent cation chelator, and a buffer. The assay of the invention further comprises polyethylene glycol as solubilizing agent. The catechol dye acts as a chemical borate sensor. The chemical borate sensor changes its optical properties upon binding to borate. The multivalent cation chelator binds multivalent cations present in a sample being analyzed. The buffer prevents changes in pH. In some embodiments, the buffer displays a solubility in water greater than 200 g/L. In particular embodiments, the solubilizing agent increases the solubility of the dye, multivalent cation chelator, and/or the buffer. In one embodiment, the operable pH range for borate concentration determination is from about 6 to about 8. In other embodiments, the operable pH range for borate concentration determination is from about 4 to about 12. The borate concentration is measureable in waters with high total dissolved solids. The assay of the present invention is defined in claim 1. The kit of the invention is defined in claim 6.

In some embodiments, the catechol dye is Alizarin Red S. In some embodiments, the multivalent cation chelator is EDTA. In some embodiments, the buffer is imidazole. In preferred embodiments, EDTA binds metals present in a sample being analyzed. In other embodiments, EDTA minimizes errors in measured borate concentration. In yet other embodiments, Alizarin Red S reacts with borate to form complex 1-BO4. In some embodiments, the borate concentration is measureable in waters with high total dissolved solids.

According to the present invention the solubilizing agent is polyethylene glycol, which is present in a range of from 1% to 10%.

The assay solution may include solution dispensed into multi-well plates. The assay solution of the invention includes freeze-dried solution. A kit for the determination of dissolved borate concentration comprising a catechol dye, a multivalent cation chelator, a solubilizing agent, and a buffer in a container containing multiple test locations, preferably a 96-well plate is described. The kit comprises the assay solution described. The present invention also relates to a method of determining the borate concentration in water, comprising contacting the sample with any one the assay solutions of the present invention. In some embodiments, the method comprises employing the inventive assay solution and a fluorometric detector. In some embodiments, the sample for determination of borate concentration is an aqueous sample. Other non-limiting types of samples for which borate concentration may be determined include soils and other solids, gels, slurries, suspensions, tissues and the like.

One skilled in the art recognizes that the concentrations of different compounds will depend on the detector. One skilled in the art recognizes that the concentrations required to obtain a linear response will vary.

The concentrations can be adjusted and the detector path length can be adjusted. For example a decrease in the path length will allow for an increase in the concentration. Increasing the path length will allow for a decrease in the concentration.

Details associated with the embodiments described above and others are presented.

### BRIEF DESCRIPTION OF THE DRAWINGS

The following drawings illustrate by way of example and not limitation. For the sake of brevity and clarity, every feature of a given structure may not be labeled in every figure in which that structure appears. Identical reference numbers do not necessarily indicate an identical structure. Rather, the same reference number may be used to indicate a similar feature or a feature with similar functionality, as may non-identical reference numbers.

Unless otherwise noted, the figures are drawn to scale, meaning that the sizes of the depicted items are accurate relative to each other for at least the embodiments depicted in the figures.
**FIG. 1A** is a drawing of the chemical reaction that occurs upon binding of Alizarin Red S to borate.
**FIG. IB** is a drawing of the sequestration of multivalent ions by coordination to EDTA.
**FIG. 1C** is a drawing of the imidazole buffering mechanism.
**FIG. 2** is a calibration curve that plots change in absorbance at 520 nm as a function of borate concentration.

### DETAILED DESCRIPTION

Various features and advantageous details are explained more fully with reference to the non-limiting embodiments that are illustrated in the accompanying drawings and detailed in the following description. It should be understood, however, that the detailed description and the specific examples, while indicating embodiments of the invention, are given by way of illustration only, and not by way of limitation. Various substitutions, modifications, additions, and/or rearrangements will become apparent to those of ordinary skill in the art from this disclosure.

The terms "a" and "an" are defined as one or more unless this disclosure explicitly requires otherwise.

The term "substantially" is defined as being largely but not necessarily wholly what is specified (and include wholly what is specified) as understood by one of ordinary skill in the art. In any disclosed embodiment, the term "substantially" may be substituted with "within [a percentage] of' what is specified, where the percentage includes .1, 1, 5, and 10 percent.

The terms "comprise" (and any form of comprise, such as "comprises" and "comprising"), "have" (and any form of have, such as "has" and "having"), "include" (and any form of include, such as "includes" and "including") and "contain" (and any form of contain, such as "contains" and "containing") are open-ended linking verbs. As a result, a composition and/or an assay solution that "comprises," "has," "includes" or "contains" one or more elements possesses those one or more elements, but is not limited to possessing only those one or more elements. Likewise, an element of a system or composition that "comprises," "has," "includes" or "contains" one or more features possesses those one or more features, but is not limited to possessing only those one or more features.

Furthermore, a structure or composition that is configured in a certain way is configured in at least that way, but may also be configured in ways that are not listed. Metric units may be derived from the English units provided by applying a conversion and rounding to the nearest millimeter.

The feature or features of one embodiment may be applied to other embodiments, even though not described or illustrated, unless expressly prohibited by this disclosure or the nature of the embodiments.

As used herein, high total dissolved solids includes values above 60,000 mg/L. In the following description, numerous specific details are provided to provide a thorough understanding of the disclosed embodiments. One of ordinary skill in the relevant art will recognize, however, that the invention may be practiced without one or more of the specific details, or with other methods, components, materials, and so forth. In other instances, well-known structures, materials, or operations are not shown or described in detail to avoid obscuring aspects of the invention.

The assay can be used as a field test for the determination of dissolved borate in aqueous solutions. In one embodiment, the assay is designed to test produced water at oil and gas sites. In particular instances, corrosive chemicals such as sulphuric acid (used in other commercially available assays) are avoided. The assay can be performed in any aqueous solution. In one embodiment of the present invention, the assay was performed in waters with extremely high total dissolved solids (TDS), where a large percentage of the TDS are multivalent metals such as, but not limited to, Ca⁺², Mg⁺², Fe⁺², and Fe⁺³.

The assay comprises a solution as made up of a catechol dye, a multivalent cation chelator and a buffer. Any catechol dye known to those of skill in the art may be used. Examples of such catechol dyes comprise Alizarin Red S and pyrocatechol violet. The multivalent cation chelator may comprise EDTA, 1,2-bis(o-aminophenoxy)ethane-N,N,N',N'-tetraacetic acid (BAPTA), Quin-2, BAPTA-AM, Fura-1, Fura-2, Fura-3, 1,2-Bis(2-Aminophenoxy)ethane-N,N,N',N'-tetraacetic acid, APTRA, 5F-APTRA, 2-Hydroxyisoquinoline-1,3(2H,4H)-dione, other different salts thereof, or any multivalent cation chelator known to those of skill in the art. Buffers may comprise imidazole, phosphate, HPEES, citrate, or other buffers known to those of skill in the art.

In some embodiments, the catechol dye exhibits a maximal borate sensitivity at a given pH. In some embodiments, a chelator is employed such that the chelator pKa is below the catechol dye borate maximal sensitivity pH. In a particular embodiment, the dye is Alizarin Red S and exhibits a maximal sensitivity at a pH of about 7.2. In a further embodiment, a metal chelator that blocks metal ions from binding to/and or interfering with Alizarin Red S has a pKa below 7.2. In this particular embodiement, the chelator is BAPTA (CAS number 85233-19-8). However, other chelators such as Fura-2 (CAS 112694-64-7) may also be used.

In preferred embodiments, a chelator is employed that is fully deprotonated at the pH necessary for the dye to be sensitive. If a chelator is protonated at a pH of interest, as in the case of EDTA at pH 7.2, the addition of metals may result in the chelator releasing protons, thereby causing the pH to change and/or necessitate the use of very high buffer concentrations. A change in pH may cause the assay to lose accuracy because the dye may change color in response to pH in a similar manner to how it changes color in response to borate. To stop this change, buffer can be added. However, the amount of buffer that can be added is limited by the solubility of the buffer. Although the solubility of buffers varies, it is preferred to use a buffer concentration of less than or equal to approximately 1M.

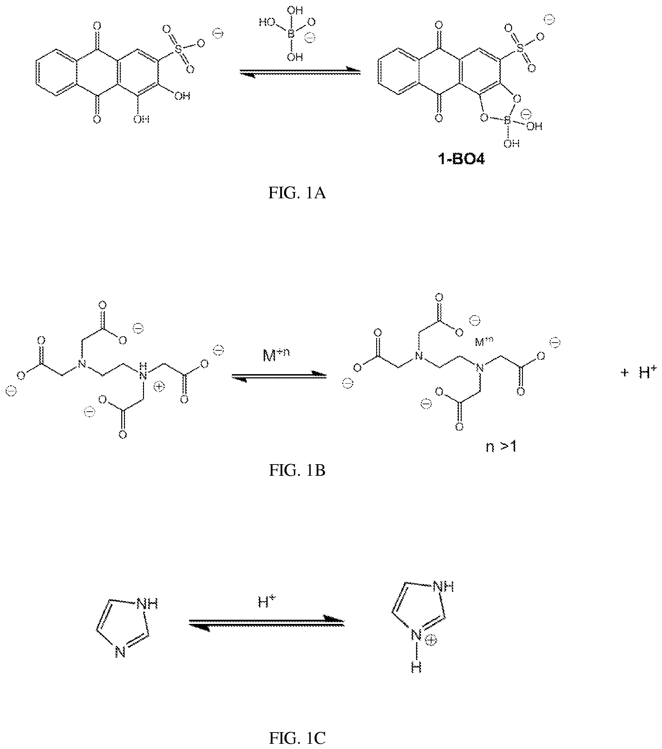

In a preferred embodiment, the assay comprises a solution as made up of compound 1 (Alizarin Red S), compound 2 (EDTA), compound 3 (imidazole) and hydroxypropyl β-cyclodextrin in water at a pH between 6 and 8 **(****FIGS. 1A-1C****).** Alizarin Red S is the chemical sensor and it changes its optical properties when it reacts with borate to form complex 1-BO4, which allows for the development of calibration curves for use in the determination of the concentration of borate in samples with unknown concentration. EDTA is a masking agent and binds any metals present in the high TDS sample being analyzed. The EDTA is important because metals can also bind to Alizarin Red S and change its optical properties, which can result in errors in the determination of the concentration of boron in unknown samples. Imidazole is the buffer. The buffer is important because Alizarin Red S changes its optical properties in response to pH. Hydroxypropyl β-cyclodextrin increases the solubility of the dye. One skilled in the art recognizes that Alizarin S can work in a variety of pH ranges, including from 1-12. In a particular embodiment, a range of 6-8 has been found to be useful. Thus, without a buffer, the pH would change on the addition of a sample, resulting in an increase in error for the assay.

In one embodiment, the assay is dispensed into 96-well plates and freeze-dried. The freeze-drying allows the assay to rapidly dissolve on the addition of a sample for analysis. Freeze dried samples are hygroscopic; thus, the 96-well plates with the freeze-dried assay are stored in mylar bags filled with nitrogen and containing a dessicant. Hydroxypropyl β-cyclodextrin prevents the dye from precipitating out of solution when temperature decreases.

The assay can be conducted as a single assay in an appropriate container. It is advantageous to be able to have a container that allows multiple testing at the same time. One skilled in the art recognizes that multiple well containers are well known in the art and can be used for multiple tests. In one embodiment, a 96-well plate is used. The assay solution includes solution dispensed into 96-well plates. The assay solution includes freeze-dried solution. A kit for the determination of dissolved borate concentration comprising a catechol dye, a multivalent cation chelator, and a buffer in a container containing multiple test locations, preferably a 96-well plate is described. The kit comprises the assay solution described. A method of determining the borate concentration in water, comprising employing the assay solution is described.

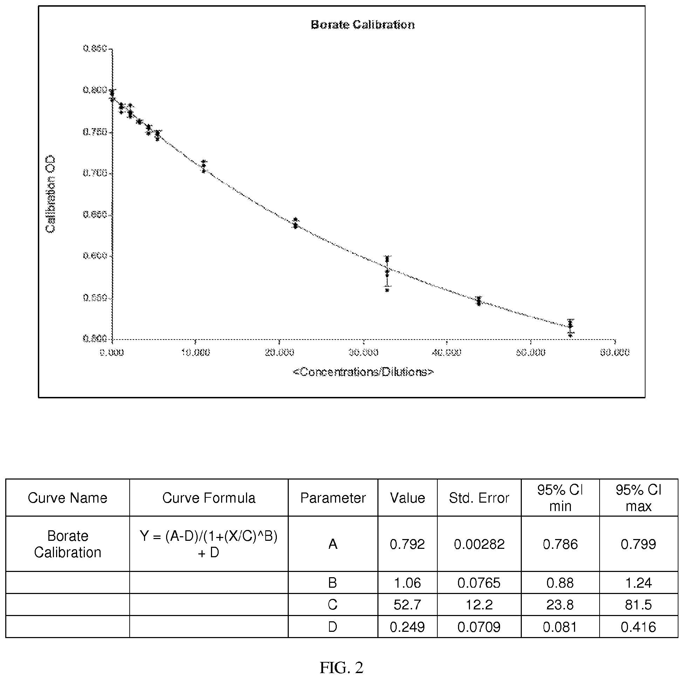

One skilled in the art recognizes that the absorbance can be read in between 200 and 620 nm. In one particular embodiment, it is found that 520 nm works well and thus, calibration curves were developed between 0 and 60 mg/L of borate by plotting the change in the absorbance at 520 nm as a function of the change in borate concentration. The resulting calibration data was fit with a curve using nonlinear regression in Gen5 software **(****FIG. 2****).** The calibration curve is used for determining the concentration of borate in samples of unknown concentration.

Any optical reader can be used to detect the results of the assay. Most optical readers will have their own software package to help normalize the curves. In one aspect of the present invention, the optical data for the assay is collected using a commercially available plate reader from Biotek. The Biotek plate reader comes with a software package called Gen5. The Gen5 software is programmed so that a user can click a button to start an experiment. Once the experimented is started, the program automatically reads the wavelength of the assay at 520 nm and plots the absorbance value on the calibration curve to determine the concentration of the unknown sample.

### EXAMPLES

The sensor solution was prepared by combining 185.5 g disodium ethylenediaminetetraacetate (EDTA) dihydrate with 69.326 g imidazole free base in 700 mL distilled water. The solution was heated until all constituents went into solution, and the pH verified to be 7.19. From a concentrated solution of Alizarin Red S (58.12 mM in distilled water), 10.32 mL were added. A large portion of the dye appeared to precipitate, but returned to solution with gentle heating. A second batch of the sensor buffer solution was prepared as above, using 185.468 g disodium EDTA dihydrate and 70.582 g imidazole free base. Both solutions were then transferred to a 2000 mL volumetric flask and diluted to the mark with distilled water, resulting in the final sensor solution: 0.6 mM ARS, 1.0 M imidazole, 0.5 M EDTA, pH 7.2.

**Table 1.**

| Well ID | Name | Well | Conc/Dil | A520 | Count | Mean | Std Dev | CV (%) |
|---|---|---|---|---|---|---|---|---|
| STD1 | Boron Standards | A1 | 0 | 0.789 | 4 | 0.796 | 0.005 | 0.652 |
| | | B1 | 0 | 0.8 | | | | |
| | | C1 | 0 | 0.8 | | | | |
| | | D1 | 0 | 0.796 | | | | |
| STD2 | Boron Standards | A2 | 1.1 | 0.774 | 4 | 0.78 | 0.004 | 0.538 |
| | | B2 | 1.1 | 0.78 | | | | |
| | | C2 | 1.1 | 0.784 | | | | |
| | | D2 | 1.1 | 0.781 | | | | |
| STD3 | Boron Standards | A3 | 2.19 | 0.769 | 4 | 0.774 | 0.006 | 0.785 |
| | | B3 | 2.19 | 0.783 | | | | |
| | | C3 | 2.19 | 0.773 | | | | |
| | | D3 | 2.19 | 0.772 | | | | |
| STD4 | Boron Standards | A4 | 3.29 | 0.761 | 4 | 0.763 | 0.002 | 0.224 |
| | | B4 | 3.29 | 0.763 | | | | |
| | | C4 | 3.29 | 0.765 | | | | |
| | | D4 | 3.29 | 0.764 | | | | |
| STD5 | Boron Standards | A5 | 4.38 | 0.749 | 4 | 0.755 | 0.004 | 0.523 |
| | | B5 | 4.38 | 0.756 | | | | |
| | | C5 | 4.38 | 0.758 | | | | |
| | | D5 | 4.38 | 0.756 | | | | |
| STD6 | Boron Standards | A6 | 5.48 | 0.742 | 4 | 0.748 | 0.004 | 0.56 |
| | | B6 | 5.48 | 0.75 | | | | |
| | | C6 | 5.48 | 0.751 | | | | |
| | | D6 | 5.48 | 0.75 | | | | |
| STD7 | Boron Standards | A7 | 10.95 | 0.703 | 4 | 0.71 | 0.005 | 0.709 |
| | | B7 | 10.95 | 0.711 | | | | |
| | | C7 | 10.95 | 0.715 | | | | |
| | | D7 | 10.95 | 0.711 | | | | |
| STD8 | Boron Standards | A8 | 21.9 | 0.636 | 4 | 0.639 | 0.004 | 0.606 |
| | | B8 | 21.9 | 0.645 | | | | |
| | | C8 | 21.9 | 0.638 | | | | |
| | | D8 | 21.9 | 0.639 | | | | |
| STD9 | Boron Standards | A9 | 32.85 | 0.56 | 4 | 0.583 | 0.018 | 3.116 |
| | | B9 | 32.85 | 0.596 | | | | |
| | | C9 | 32.85 | 0.599 | | | | |
| | | D9 | 32.85 | 0.577 | | | | |
| STD10 | Boron Standards | A10 | 43.8 | 0.543 | 4 | 0.548 | 0.004 | 0.753 |
| | | B10 | 43.8 | 0.545 | | | | |
| | | C10 | 43.8 | 0.551 | | | | |
| | | D10 | 43.8 | 0.551 | | | | |
| STD11 | Boron Standards | A11 | 54.75 | 0.505 | 4 | 0.516 | 0.008 | 1.478 |
| | | B11 | 54.75 | 0.519 | | | | |
| | | C11 | 54.75 | 0.522 | | | | |
| | | D11 | 54.75 | 0.519 | | | | |

Table Concentrations are in mg/L.

## Claims

1. An assay for determining borate concentration, the assay comprising a catechol dye, a multivalent cation chelator, polyethylene glycol as solubilizing agent, and a buffer, wherein the assay comprises a freeze-dried solution and wherein the solubilizing agent is present in an amount ranging from 1% to 10%.

2. The assay of claim 1, wherein the catechol dye is Alizarin Red S.

3. The assay of any one of the preceding claims, wherein the multivalent cation chelator is EDTA.

4. The assay of any one of the preceding claims, wherein the buffer is imidazole.

5. The assay of any one of the preceding claims, wherein the operable pH range is from about 6.9 to about 7.4.

6. A kit comprising the assay of any one of the preceding claims.

7. A method of determining the borate concentration of a sample, the method comprising contacting the sample with any one of the assays of claims 1 to 5, and determining the concentration of borate in the sample, wherein the sample is an aqueous sample.

8. The method of claim 7, further comprising employing a fluorometric detector.

9. The method of claim 7 or 8, wherein the method is used to test produced water at oil and gas sites.

10. The method of claim 9, wherein the method is used to test waters with high total dissolved solids, wherein high total dissolved solids include values above 60,000 mg/L, and wherein preferably a large percentage of the total dissolved solids are multivalent metals including Ca⁺², Mg⁺², Fe⁺², and Fe⁺³

11. The method of any one of claims 7 to 9, wherein the method comprises developing calibration curves between 0 and 60 mg/L of borate by plotting the change in the absorbance at 520 nm as a function of the change in borate concentration.

12. The method of any one of claims 7 to 10, wherein the solution resulting from contacting the assay with the sample is dispensed into multi-well plates.

13. The method of claim 12, wherein the multi-well plate is a 96-well plate.

## Patentansprüche

1. Assay zur Bestimmung der Konzentration von Borat, wobei der Assay einen Catecholfarbstoff, einen mehrwertigen Kationchelatbildner, Polyethylenglycol als Löslichkeitsvermittler und ein Puffersystem umfasst, wobei der Assay eine gefriergetrocknete Lösung umfasst und wobei der Löslichkeitsvermittler in einer Menge im Bereich von 1 % bis 10 % vorhanden ist.

2. Assay nach Anspruch 1, wobei der Catecholfarbstoff Alizarin Rot S ist.

3. Assay nach einem der vorhergehenden Ansprüche, wobei der mehrwertige Kationchelatbildner EDTA ist.

4. Assay nach einem der vorhergehenden Ansprüche, wobei der Puffer Imidazol ist.

5. Assay nach einem der vorhergehenden Ansprüche, wobei der durchführbare pH-Bereich von etwa 6,9 bis etwa 7,4 beträgt.

6. Kit, der den Assay gemäß einem der vorhergehenden Ansprüche umfasst.

7. Verfahren zur Bestimmung der Konzentration von Borat einer Probe, bei dem die Probe mit einem der Assays gemäß einem der Ansprüche 1 bis 5 kontaktiert wird und die Konzentration an Borat in der Probe bestimmt wird, wobei die Probe eine wässrige Probe ist.

8. Verfahren nach Anspruch 7, bei dem ein fluorometrischer Detektor verwendet wird.

9. Verfahren nach Anspruch 7 oder 8, bei dem das Verfahren verwendet wird, um an Öl- und Gasförderstellen anfallendes Wasser zu bestimmen.

10. Verfahren nach Anspruch 9, bei dem das Verfahren verwendet wird, um Wässer mit einem hohen Gesamtgehalt an gelösten Feststoffen zu testen, wobei hohe Gesamtgehalte an gelösten Feststoffen Werte oberhalb 60.000 mg/l einschließen und wobei vorzugsweise ein großer Prozentsatz der insgesamt gelösten Feststoffe mehrwertige Metalle sind, einschließlich Ca⁺², Mg⁺², Fe⁺² und Fe⁺³.

11. Verfahren nach einem der Ansprüche 7 bis 9, bei dem Kalibrierungskurven zwischen 0 und 60 mg Borat entwickelt werden, indem die Änderung in der Extinktion bei 520 nm als Funktion der Änderung in der Boratkonzentration geplottet werden.

12. Verfahren nach einem der Ansprüche 7 bis 10, bei dem die Lösung, die aus dem Kontaktieren des Assays mit der Probe resultiert, in Mikrotiterplatten gegeben werden.

13. Verfahren nach Anspruch 12, bei dem die Mikrotiterplatte eine Platte mit 96 Näpfchen ist.

## Revendications

1. Dosage pour déterminer la concentration de borate, le dosage comprenant un colorant catéchol, un chélateur de cations multivalents, du polyéthylèneglycol en tant qu'agent solubilisant, et un tampon, où le dosage comprend une solution lyophilisée et où l'agent solubilisant est présent en une quantité située dans la plage allant de 1 % à 10 %.

2. Dosage selon la revendication 1, dans lequel le colorant catéchol est le rouge d'alizarine S.

3. Dosage selon l'une quelconque des revendications précédentes, dans lequel le chélateur de cations multivalents est l'EDTA.

4. Dosage selon l'une quelconque des revendications précédentes, dans lequel le tampon est l'imidazole.

5. Dosage selon l'une quelconque des revendications précédentes, dans lequel la plage de pH utilisable va d'environ 6,9 à environ 7,4.

6. Kit comprenant le dosage de l'une quelconque des revendications précédentes.

7. Procédé pour déterminer la concentration de borate dans un échantillon, le procédé comprenant la mise en contact de l'échantillon avec l'un quelconque des dosages des revendications 1 à 5, et la détermination de la concentration de borate dans l'échantillon, dans lequel l'échantillon est un échantillon aqueux.

8. Procédé selon la revendication 7, comprenant en outre l'emploi d'un détecteur fluorométrique.

9. Procédé selon la revendication 7 ou 8, où le procédé est utilisé pour tester l'eau produite sur des sites de pétrole et de gaz.

10. Procédé selon la revendication 9, où le procédé est utilisé pour tester de l'eau ayant une forte teneur totale en solides dissous, dans lequel la forte teneur totale en solides dissous comprend des valeurs supérieures à 60 000 mg/l, et dans lequel de préférence un pourcentage important des solides dissous totaux est constitué de métaux multivalents comprenant Ca⁺², Mg⁺², Fe⁺² et Fe⁺³.

11. Procédé selon l'une quelconque des revendications 7 à 9, où le procédé comprend le développement de courbes d'étalonnage entre 0 et 60 mg/l de borate par tracé du changement de l'absorbance à 520 nm en fonction du changement de la concentration de borate.

12. Procédé selon l'une quelconque des revendications 7 à 10, dans lequel la solution résultant de la mise en contact du dosage avec l'échantillon est distribuée dans des plaques à puits multiples.

13. Procédé selon la revendication 12, dans lequel la plaque à puits multiples est une plaque à 96 puits.
